(19) Europäisches Patentamt

European Patent Office

Office européen des brevets

(11) Veröffentlichungsnummer: **0 537 497 A2**

## EUROPÄISCHE PATENTANMELDUNG

(21) Anmeldenummer: **92116008.1**

(22) Anmeldetag: **18.09.92**

(51) Int. Cl.5: **C12P 21/08**, C12N 5/20,
G01N 33/577, G01N 33/569

(30) Priorität: **17.10.91 DE 4134297 U**

(43) Veröffentlichungstag der Anmeldung:
**21.04.93 Patentblatt 93/16**

(84) Benannte Vertragsstaaten:
**AT BE CH DE DK ES FR GB IT LI LU NL PT SE**

(71) Anmelder: **BEHRINGWERKE**
**Aktiengesellschaft**
**Postfach 1140**
**W-3550 Marburg 1(DE)**

(72) Erfinder: **Bredt, Wolfgang**
**Sickingenstrasse 70**
**W-7800 Freiburg(DE)**
Erfinder: **Gerstenecker, Bernhard**
**Untere Vorstadt 103**
**W-7470 Albstadt 1(DE)**
Erfinder: **Jacobs, Enno**
**Schlehenrain 10**
**W-7800 Freiburg(DE)**
Erfinder: **Schuy, Wilhelm**
**Schliefelderweg 6**
**W-5431 Obererbach(DE)**

(54) **Monoclonale Antikörper gegen Mycoplasma pneumoniae, diese produzierende Hybridome, Verfahren zu deren Herstellung sowie deren Verwendung.**

(57) Die Erfindung betrifft monoclonale Antikörper und deren Fragmente, die spezifisch für Mycoplasma pneumoniae sind und eine Kreuzreaktivität von 1 % oder weniger mit anderen Spezies der Gattung Mycoplasma oder anderen Erregerspezies der Begleitflora aufweisen sowie Verfahren zur Herstellung der erfindungsgemäßen monoclonalen Antikörper. Die Erfindung betrifft ferner Hybridome, die die erfindungsgemäßen Antikörper produzieren sowie Verfahren zu deren Herstellung. Schließlich betrifft die Erfindung die Verwendung der erfindungsgemäßen monoclonalen Antikörper zum Nachweis von Mycoplasma pneumoniae in einer Probe.

EP 0 537 497 A2

Die Erfindung betrifft monoclonale Antikörper und deren Fragmente, die spezifisch für Mycoplasma pneumoniae sind und eine Kreuzreaktivität von 1 % oder weniger mit anderen Spezies der Gattung Mycoplasma oder anderen Erregerspezies der Begleitflora aufweisen sowie Verfahren zur Herstellung der erfindungsgemäßen monoclonalen Antikörper. Die Erfindung betrifft ferner Hybridome, die die erfindungsgemäßen Antikörper produzieren sowie Verfahren zu deren Herstellung. Schließlich betrifft die Erfindung die Verwendung der erfindungsgemäßen monoclonalen Antikörper zum Nachweis von Mycoplasma pneumoniae in einer Probe.

Mycoplasma pneumoniae (M. pneumoniae) verursacht Erkrankungen des oberen und des unteren Respirationstraktes. Da M. pneumoniae im Gegensatz zu Viren gegen Antibiotika empfindlich ist, hat eine schnelle Diagnose erhebliche therapeutische Konsequenzen. Eine sichere Abgrenzung einer M. pneumoniae-Infektion von viralen bzw. bakteriellen Infektionen mit ähnlichen klinischen Symptomen war jedoch bisher nicht möglich. Ebenso war die verfügbare Labordiagnostik zur Bestimmung von M. pneumoniae bisher unbefriedigend. Der Erregernachweis durch Kultivierung hat wegen des sehr langen Wachstums auf anspruchsvollen Medien (1 bis 2 Wochen) nur bestätigenden Wert. In den meisten Fällen wird derzeit die Diagnose serologisch gestellt. Der gebräuchlichste Test, die Komplementbindungsreaktion (KBR), verwendet einen Glykolipidextrakt aus M. pneumoniae als Antigen. Antikörper gegen dieses Glykolipid zeigen jedoch Kreuzreaktionen sowohl mit Bakterien (Streptococcus MG intermedicus) als auch mit Pflanzenlipiden oder Bestandteilen menschlicher Zellen und führen damit zu falsch positiven Reaktionen. Insbesondere bei Erkrankungen, die mit Zellzerfall einhergehen, z.B. Pankreatitis, Meningitis und Carditis, sind derartige falschpositiven KBR-Reaktionen beschrieben worden. Andere verfügbare Testverfahren sind für den Routinenachweis von M. pneumoniae ungeeignet, da sie entweder zu arbeitsaufwendig (z.B. Immunfluoreszenz), nur in Speziallaboratorien verwendbar (Adhärenzinhibitionstest) oder ebenso wenig spezifisch wie die KBR (ELISA mit M. pneumoniae-Gesamtextrakt) sind.

Die Produktion von monoclonalen Antikörpern gegen verschiedene Proteine von M. pneumoniae ist in mehreren Publikationen beschrieben worden.

Monoclonale Antikörper gegen das P1-Protein (Adhäsin oder 168 kd-Protein), mit dem die Inhibierung der Bindung von M. pneumoniae an Erythrocyten erreicht werden konnte, wurden etabliert (B. Gerstenecker und E. Jacobs, Journal of General Microbiology 136 (1990), 471).

In einer Veröffentlichung von E. Jacobs et al. (Diagnostik von Infektionskrankheiten, Herausgeber: Projektträgerschaft Forschung im Dienste der Gesundheit in der Deutschen Forschungs- und Versuchsanstalt für Luft- und Raumfahrt e.V., Band 1, (1987), 84) werden 2 monoclonale Antikörper gegen das Adhäsin beschrieben. Beide (moAK 14 C11, moAK 11 G7) reagieren nur in Dot-Blot-Verfahren mit M. pneumoniae, nicht aber in anderen immunometrischen Verfahren. Darüber hinaus zeigt moAK 14 C11 Kreuzreaktionen mit M. salivarium und M. genitalium und moAK 11G7 Kreuzreaktionen mit z.B. Branhamella catarrhalis (vgl. Tabelle I).

Obwohl für die Herstellung monoclonaler Antikörper gut etablierte Verfahren zur Verfügung stehen, bestehen oft noch beträchtliche Schwierigkeiten, Hybridomzellinien zu produzieren und zu selektionieren, die ausschließlich bestimmte vorteilhafte Eigenschaften besitzen.

So zeigen die vorstehend zitierten Arbeiten monoclonale Antikörper, die entweder mit einer oder mehreren Mycoplasmaspezies oder mit anderen Erregerspezies der Begleitflora, wie z.B. H. influenzae N. meningitidis, S.pneumoniae oder P. aeruginosa in den zu untersuchenden biologischen Proben kreuzreagieren, bei der Kreuzreaktivität nicht ausgeschlossen oder nur unter bestimmten Testbedingungen geprüft wurde.

Der vorliegenden Erfindung lag daher das technische Problem zugrunde, einen monospezifischen Antikörper herzustellen, der mit Mycoplasma pneumoniae reagiert, aber keine oder keine wesentliche Kreuzreaktivität mit anderen Mycoplasmaspezies und den vorstehend genannten anderen Erregerspezies der Begleitflora aufweist. Die Lösung dieses technischen Problems wird durch die in den Patentansprüchen gekennzeichneten Ausführungsformen erzielt.

Gegenstand der Erfindung sind somit ein Antikörper und dessen Fragmente, der (die) spezifisch für M. pneumoniae ist (sind) und eine Kreuzreaktivität von 1 % oder weniger mit anderen Spezies der Gattung Mycoplasma aufweist (aufweisen).

Der Begriff "Fragmente" bezeichnet die dem Fachmann an sich bekannten immunreaktiven Fragmente von Antikörpern. Bevorzugt unter diesen Fragmenten sind die F(ab')$_2$-Fragmente.

In einer bevorzugten Ausführungsform weist (weisen) der erfindungsgemäße monoclonale Antikörper und dessen Fragmente eine Kreuzreaktivität von 1 % oder weniger mit anderen Erregerspezies der Begleitflora auf. Der Begriff "Begleitflora" bedeutet einen oder mehrere pathogene oder apathogene Mikroorganismen, die zur gleichen Zeit und an der gleichen Stelle im Organismus vorkommen können. Der erfindungsgemäße monoclonale Antikörper weist diese vorteilhaften Spezifitätseigenschaften beispielsweise

2

in einem halbmonoclonalen Festphasen-Sandwichassay auf. Unter einem halbmonoclonalen Festphasen-Sandwichassay versteht man ein an eine Festphase gebundenen z.B. polyclonalen Fängerantikörper, der das Antigen, wie z.B. ein Protein von Mycoplasma pneumoniae, bindet und einen z.B. monoclonalen Nachweisantikörper, der das gebundene Antigen über eine folgende Indikatorreaktion nachweist. Beispiele für solche halbmonoclonalen Festphasen-Sandwichassays sind der Antigen-ELISA (Capture-ELISA) und der PI-ELISA. Der erfindungsgemäße monoclonale Antikörper ist zum Nachweis von M. pneumoniae darüber hinaus in verschiedenen, dem Fachmann an sich bekannten Verfahren, z.B. Immunoblot, Zell-ELISA, Präzipitation, indirekter EIA verwendbar.

In einer besonders bevorzugten Ausführungsform ist (sind) der erfindungsgemäße monoclonale Antikörper und dessen Fragmente spezifisch für das P1-Protein von Mycoplasma pneumoniae. Das P1-Protein ist dem Durchschnittsfachmann auch als Adhäsin oder 168-kd-Protein bekannt.

In einer weiteren bevorzugten Ausführungsform gehört die schwere Kette des erfindungsgemäßen monoclonalen Antikörpers bzw. von dessen Fragmenten der $\gamma$1-Subklasse und die leichte Kette der $\varkappa$-Klasse an.

In einer anderen bevorzugten Ausführungsform gehört die schwere Kette der $\gamma$3-Subklasse und die leichte Kette der $\varkappa$-Klasse an.

Die Bestimmung der Immunglobulinklassen bzw. Subklassen erfolgt nach dem Fachmann an sich bekannten Verfahren. Darüber hinaus lassen sich die erfindungsgemäßen Antikörper nach an sich bekannten Verfahren, z.B. mit Hinsicht auf ihr elektrophoretisches Fokusierungsmuster oder ihre Affinitätskonstante charakterisieren.

Ein weiterer Gegenstand der Erfindung ist ein Hybridom, das einen der erfindungsgemäßen monoclonalen Antikörper produziert.

In einer bevorzugten Ausführungsform des erfindungsgemäßen Hybridoms stammt die zur Fusion verwendete Milzzelle aus einer BALB/c-Maus.

In einer weiteren bevorzugten Ausführungsform des erfindungsgemäßen Hybridoms ist die zur Fusion verwendete Myelomzelle X63/Ag8.653.

Ein weiterer Gegenstand der Erfindung ist die Hybridomlinie M57.

Ein weiterer Gegenstand der Erfindung ist die Hybridomlinie M74.

Ein weiterer Gegenstand der Erfindung ist die Hybridomlinie P1.25.

Ein weiterer Gegenstand der Erfindung ist die Hybridomlinie P1.27.

Ein weiterer Gegenstand der Erfindung ist die Hybridomlinie M75.

Diese Hybridome stammen aus zwei verschiedenen Fusionen, für die Myelomzellen der Linie X63/Ag8.653 und Milzzellen von BALB/c-Mäusen, die entweder mit M. pn-Zellen (Fusion M) oder gereinigten P1-Protein (Fusion P1) immunisiert worden waren, verwendet worden waren. Alle 5 Hybridomlinien haben 1 Jahr lang in Zellkultur stabile Antikörper einer Spezifität sezerniert. Die von allen fünf Hybridomen sezernierten Antikörper sind spezifisch für das P1-Protein; keiner dieser Antikörper zeigt eine signifikante Kreuzreaktion mit anderen Mycoplasmaspezies, wie M. genitalium, M. hominis, M. fermentans, M. salivarium oder M. orale. Diese Mycoplasmaspezies können mit M. pneumoniae in der Begleitflora vorkommen und die unzweideutige Diagnose von M. pneumoniae im Stand der Technik erschweren.

Die Hybridome wurden am 11.4.1991 und am 24.5.1991 beim PHLS Centre for Applied Microbiology & Research Porton Down, Salisbury, Wilts. SP4OJG, England unter den Nummern 91041010 (M57), 91041011 (M74), 91041012 (P1.25), 91041013 (P1.27) und 91052310 (M75) hinterlegt.

Ein weiterer Gegenstand der Erfindung ist ein Kit zum Nachweis von Mycoplasma pneumoniae in einer Probe, der mindestens einen der erfindungsgemäßen monoclonalen Antikörper enthält. Der erfindungsgemäße Kit beruht vorzugsweise auf dem Fachmann an sich bekannten immunchemischen heterogenen oder homogenen Bestimmungsmethoden, wobei bei den homogenen Verfahren die Partikel-verstärkte Nephelometrie oder Turbidimetrie bevorzugt ist.

In einer besonders bevorzugten Ausführungsform des erfindungsgemäßen Kits erfolgt der Nachweis von M. pneumoniae durch ein festphasenimmunometrisches System. Festphasenimmunometrische Systeme werden bei den heterogenen Immunoassays bevorzugt eingesetzt. Ein bevorzugtes Beispiel für einen heterogenen Immunoassay ist der festphasengebundene Sandwichassay, wobei die Festphase bevorzugterweise ein Polystyrolröhrchen, eine Mikrotiterplatte, ein Latexpartikel, ein magnetisierbares Partikel oder eine flächenförmige Festphase ist.

Ein weiterer Gegenstand der Erfindung ist ein Verfahren zur Herstellung eines erfindungsgemäßen Hybridoms, wobei man

(a) eine Maus mit Mycoplasma pneumoniae-Zellen oder dem P1-Protein von Mycoplasma pneumoniae immunisiert,

(b) Milzzellen aus der immunisierten Maus mit geeigneten Myelomzellen fusioniert; und

(c) die im Schritt (b) erhaltenen Hybridome auf Sezernierung von Antikörpern, die spezifisch für M. pneumoniae sind und eine Kreuzreaktivität von 1 % oder weniger mit anderen Spezies der Gattung Mycoplasma aufweisen selektioniert.

Ein weiterer Gegenstand der Erfindung ist ein Verfahren zur Herstellung eines erfindungsgemäßen Hybridoms, wobei man

(a) eine Maus mit Mycoplasma pneumoniae-Zellen oder dem P1-Protein von Mycoplasma pneumoniae immunisiert,

(b) Milzzellen aus der immunisierten Maus mit geeigneten Myelomzellen fusioniert;

(c) die im Schritt (b) erhaltenen Hybridome auf Sezernierung von Antikörpern, die spezifisch für M. pneumoniae sind und eine Kreuzreaktivität von 1 % oder weniger mit anderen Spezies der Gattung Mycoplasma aufweisen selektioniert; und

(d) die im Schritt (c) erhaltenen Hybridome auf Sezernierung von Antikörpern, die eine Kreuzreaktivität von 1 % oder weniger mit anderen Erregerspezies der Begleitflora aufweisen selektioniert.

Die erfindungsgemäßen Hybridome werden hergestellt, indem Mäuse mit einem geeigneten Immunogen (M. pneumoniae-Zellen oder P1-Protein) nach an sich bekannten Verfahren (vgl. z. B. Harlow und Lane, "Antibodies", Cold Spring Harbor Laboratory, Cold Spring Harbor, 1988) immunisiert werden.

In einer bevorzugten Ausführungsform sind die für die Immunisierung verwendeten Mäuse BALB/c-Mäuse. Die Immunisierungen werden im Abstand von mindestens einer Woche durchgeführt. Vorzugsweise werden die Mäuse mindestens 2 mal immunisiert, wobei die letzte Immunisierung vorzugsweise intravenös und/oder intraperitoneal erfolgt. Der Antikörpertiter wird nach jeder Immunisierung überprüft. Dazu wird den Mäusen 3 bis 5 Tage nach der Injektion des Immunogens eine Blutprobe entnommen, Serum davon nach an sich bekannten Verfahren gewonnen und auf den Titer von M. pneumoniae- oder P1-spezifischen Antikörpern getestet. Bei einem ausreichend hohen Titer werden die Mäuse getötet, die Milzen entnommen und die einzelnen Milzzellen unter sterilen Bedingungen aus dem Gewebe gelöst. Die Milzzellen werden nach ein- oder mehrmaligem Waschen mit Zellen einer Maus-Myelomlinie fusioniert.

In einer bevorzugten Ausführungsform ist die zur Fusion verwendete Myelomlinie X63/Ag 8.653. Vorzugsweise wird bei der Fusion ein Verhältnis von Milz- zu Myelomzellen von 2:1 bis 10:1 verwendet. Als Fusionspromotor dient vorzugsweise Polyethylenglykol. Es stehen jedoch auch andere Fusionspromotoren, wie z.B. das Sendaivirus zur Verfügung.

Nach der Fusioniervng werden die Zellen auf Mikrotitrationsplatten, vorzugsweise auf Platten mit 96 Vertiefungen, ausgesät. Die Mikrotitrationsplatten sind vorzugsweise am Tag zuvor mit Makrophagen oder Thymocyten aus syngenen oder congenen Mäusen versetzt worden, die das Wachstum von Hybridomen fördernde Faktoren, z.B. Interleukin 6 sezernieren.

Als Wuchsmedium wird ein Selektivmedium, vorzugsweise HAT-Medium verwendet. Die Milzzellen sind in Zellkultur nur begrenzt lebensfähig und sterben nach wenigen Zellteilungen ab. Die als Fusionspartner gewählte Myelomzellinie weist einen Defekt in einem Enzym, das eine Rolle in der Nucleotidsynthese spielt (z.B. der Hypoxanthin-Guanin-Phosphoribosyl-Transferase) auf und ist dadurch in HAT-Medium nicht lebensfähig. Nur Hybridome, die von der Milzzelle den intakten enzymatischen Apparat und von der Myelomzelle die Fähigkeit zum unbegrenzten Wachstum in Zellkultur erhalten haben, überleben in dem Selektionsmedium.

Die Vertiefungen der Mikrotitrationsplatten werden nach 1 bis 2 Wochen auf Wachstum von Hybridomen untersucht. Das Wuchsmedium von Hybridom-positiven Vertiefungen wird anschließend auf die Anwesenheit von Antikörpern mit den gewünschten Eigenschaften geprüft. Das Testsystem ist dabei in der Regel auf die Eigenschaften des Immunogens bzw. Antigens abgestellt.

Hybridome, die Antikörper mit den gewünschten Eigenschaften sezernieren, werden nach dem Verfahren der limitierenden Verdünnung mindestens einmal subcloniert, um ihre Clonalität zu sichern. Üblicherweise verdünnt man die Zellen soweit, daß statistisch gesehen 0,5 bis 3 Zellen pro Vertiefung wachsen. Darüber hinaus hat sich das Verfahren der Subclonierung vorteilhaft bei der Herstellung von Hybridomen mit stabilen Eigenschaften erwiesen. Die Verfahren zur Herstellung von Hybridomen sind detailliert beschrieben z.B. in Harlow und Lane, a.a.O., Melchers, Potter und Warner, Herausgeber, "Lymphocyte Hybridomas", Springer-Verlag, Berlin, 1979 und in J.H. Peters und H. Baumgarten, Monoclonale Antikörper, Herstellung und Charakterisierung, Springer-Verlag, Berlin 1990.

Ein weiterer Gegenstand der Erfindung ist die Herstellung eines erfindungsgemäßen monoclonalen Antikörpers, wobei man ein erfindungsgemäßes Hybridom

(a) in vitro züchtet; und

(b) den monoclonalen Antikörper aus dem Kulturmedium gewinnt.

Ein weiterer Gegenstand der Erfindung ist die Herstellung eines erfindungsgemäßen monoclonalen Antikörpers, wobei man ein erfindungsgemäßes Hybridom

(a) in vivo in der Peritonealkavität einer syngenen oder congenen Maus züchtet; und

(b) den monoclonalen Antikörper aus der Ascitesflüssigkeit gewinnt.

Größere Mengen der erfindungsgemäßen monoclonalen Antikörper können durch dem Fachmann an sich bekannte Verfahren durch in vitro-Zellkultur der erfindungsgemäßen Hybridome und nachfolgende Reinigung und Gewinnung aus dem Überstand oder durch in vivo-Züchtung der Hybridome in der Peritonealkavität congener oder syngener Mäuse und nachfolgender Reinigung der Antikörper aus der Ascitesflüssigkeit gewonnen werden (vgl. Harlow und Lane, a.a.O.).

Ein weiterer Gegenstand der Erfindung ist die Verwendung eines erfindungsgemäßen monoclonalen Antikörpers zum Nachweis von Mycoplasma pneumoniae in einer Probe.

Die Verwendung der erfindungsgemäßen monoclonalen Antikörper kann im Rahmen eines diagnostischen Verfahrens erfolgen, wobei mindestens einer der Antikörper als spezifischer Bindungspartner des M. pneumoniae-Antigens eingesetzt wird. Der zweite spezifische Bindungspartner kann ein weiterer Antikörper oder ein Antikörperfragment, ein Lectin oder ein Rezeptor sein. Bevorzugt ist die Verwendung der erfindungsgemäßen monoclonalen Antikörper in einem diagnostischen Verfahren, vorzugsweise in einem Einschritt-Assay, bei dem auch der zweite spezifische Bindungspartner ein erfindungsgemäßer monoclonaler Antikörper ist, der ein anderes Epitop erkennt, als der erste spezifische Bindungspartner.

Zum Nachweis und zur Quantifizierung kann dabei einer der spezifischen Bindungspartner eine nachweisbare Markierung tragen. Diese Markierungen sind dem Fachmann an sich bekannt und können z.B. ein Chromophor, ein Luminophor, ein Fluorophor, ein Enzym, ein radioaktives Isotop oder ein gefärbtes oder ungefärbtes Partikel sein.

Zur Herstellung Antikörper-beschichteter Festphasen sind Verfahren bevorzugt, die die nicht-markierten spezifischen Bindungspartner nach dem Fachmann an sich bekannten Verfahren direkt oder indirekt, z.B. über einen weiteren Antikörper oder eine Biotin-Avidin-Brücke, die an eine Festphase gekoppelt ist, binden.

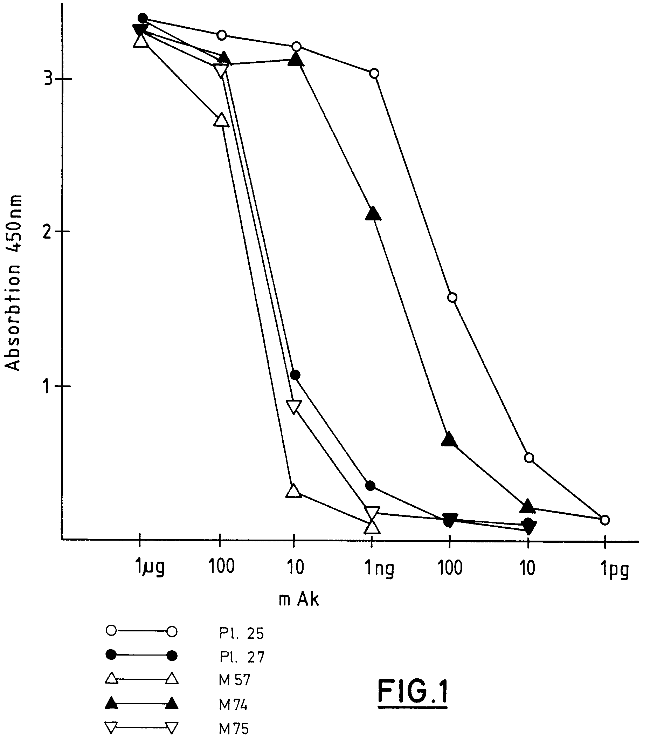

Figur 1 zeigt die Avidität der von den Hybridomen M57, M74, P1.25, P1.27 und M75 sezernierten monoclonalen Antikörper für Mycoplasma pneumoniae anhand von Standardverdünnungskurven, vgl. Beispiel 6.

Die Beispiele erläutern die Erfindung.

**Beispiel 1**

**Halbmonoclonaler Doppelsandwich-Test**

A. Herstellung von Fängerantikörpern

Kaninchen (Kreuzung aus Deutschem Riesen und Großem Chinchilla) wurden mit 0,5 mg M. pneumoniae-Protein, wie von T. W. Kok et al. in Epidem. Inf. 101 (1988), 669 beschrieben, immunisiert. Die IgG-Fraktion des Immunserums wurde durch Gelpermeationschromatographie gereinigt. Das Serum wurde dazu auf eine Sephacryl® S-300 Superfine-Säule (Pharmacia), äquilibriert mit 50 mM Tris/HCl (pH 8,0) aufgetragen und die IgG-Fraktion mit 500 mM NaCl eluiert.

B. Beschichtung von Mikrotitrationsplatten mit Kaninchen-Antikörpern

Mikrotitrationsplatten mit 96 Vertiefungen der Firma NUNC (Roskilde, Dänemark) wurden mit gereinigtem Kaninchen anti-M. pneumoniae-Antikörpern (vgl. vorstehend A), wie in der europäischen Patentanmeldung 89103478 beschrieben, beschichtet.

C. Durchführung des halbmonoclonalen Doppelsandwich-Test (Capture Assay)

In einer Mikrotitrationsplatte, beschichtet wie unter B beschrieben, wurden 0,1 ml mit Proben-Puffer STD (Produkt Nr.: OUWO; Behringwerke AG, Marburg, BRD) solubilisierte Zellen von M. pneumoniae und anderen "Nicht-Mycoplasma-pneumoniae-Spezies" der Begleitflora (verschiedene Verdünnungen von 1 µg bis 0,4 ng in Proben-Puffer STD) 1 Stunde bei 37°C inkubiert. Danach wurde dreimal mit 0,3 ml Waschlösung POD (Produkt Nr.: OSEW, Behringwerke AG, Marburg, BRD) gewaschen. 0,1 ml pro Vertiefung der Mikrotitrationsplatte des zu testenden monoclonalen Antikörpers (vgl. Tabelle I), verdünnt in Konjugat-Puffer Microbiol. (Produkt Nr.: OUWW, Behringwerke AG, Marburg, BRD) wurden 30 Minuten bei 37°C inkubiert. Danach wurde dreimal, wie oben beschrieben, gewaschen. Anschließend wurde das Testsystem mit 0,1 ml eines anti-Maus-IgG/POD-Konjugats (Produkt Nr.: NCIK03, Behringwerke AG, Marburg, BRD), 1:8000 verdünnt in Konjugat-Puffer Microbiol., 60 Minuten bei 37°C inkubiert. Danach wurde dreimal, wie vorstehend beschrieben, gewaschen. Für die Farbentwicklung wurden 0,1 ml gebrauchsfertige Chromogenlösung TMB in jede Vertiefung pipettiert. Die Farbentwicklung wurde nach 30 Minuten Inkubation bei Raumtemperatur mit Stopplösung POD gestoppt.

Gebrauchsfertige Chromogenlösung wurde aus den Reagenzien des Kits "Zusatzreagenzien für Enzygnost®/TMB" (Produkt Nr.: OUVP, Behringwerke AG, Marburg, BRD) und nach den darin enthaltenen Anweisungen angesetzt. Stopplösung POD ist ebenfalls im vorstehend genannten Kit enthalten. Die Farbentwicklung wurde mit einem Photometer für Mikrotitrationsplatten (Behring ELISA Processor II; Bebringwerke AG, Marburg) bestimmt. Meßwellenlänge war 450 nm, als Korrekturwellenlänge wurde 650 nm verwendet.

## Beispiel 2

### Durchführung eines homogenen, polyclonalen Capture-ELISA

Die Bindung anderer Mycoplasmaarten (M. genitalium, M. fermentans, M. hominis, M. salivarium, M. orale) durch das gegen M. pneumoniae gewonnene Kaninchenserum wurde in einem homogenen polyclonalen Capture-ELISA nachgewiesen. In diesem Assay wurde das Kaninchen-Serum sowohl als an die Festphase gebundener Fänger-Antikörper als auch als sekundärer Detektor-Antikörper verwendet. Die Bindung des Detektor-Antikörpers, der nach im Stand der Technik bekannten Verfahren mit Biotin konjugiert war, wurde durch ein an sich bekanntes Avidin/POD-Konjugat nachgewiesen.

Die Versuchsdurchführung ist mit Ausnahme des Detektionssystems identisch mit der in Beispiel 1 beschriebenen für den halbmonoclonalen Doppelsandwich-Test.

## Beispiel 3

### Herstellung von monoclonalen anti-Mycoplasma pneumoniae Antikörpern

A. Vermehrung von Mycoplasma pneumoniae

M. pneumoniae Stamm FH wurde in Hayflick's modifizierten Eagles Medium (L. Hayflick in Texas Reports on Biology and Medicine 23 (1965), 285) vermehrt. Die Zellen wurden durch Zentrifugation (10 Minuten, 1000 x g) sedimentiert und zweimal in steriler Phosphat-gepufferter Kochsalzlösung (PBS) gewaschen.

B. Präparation von P1-Protein

Die Reinigung von P1-Protein aus M. pneumoniae ist in der europäischen Patentanmeldung 89105006 beschrieben.

C. Immunisierungen

Männliche BALB/c-Mäuse im Alter zwischen 6 und 8 Wochen wurden mit ca. $10^8$ Kolonie-bildenden Einheiten frisch geernteter und gewaschener, intakter M. pneumoniae-Zellen in PBS oder mit 500 $\mu$g gereinigtem P1-Protein, emulgiert in kompletten Freund'schen Adjuvans (CFA) intraperitoneal immunisiert. Für die Immunisierungen wurde das P1-Protein vor der Emulsion in CFA und nach der Aufreinigung durch Chloroform-Methanol-Extraktion von überschüssigen Detergens (SDS) abgetrennt und anschließend durch Ultraschallbehandlung in PBS resuspendiert.

Die Immunisierungen wurden 4 mal in 10-tägigen Abständen wiederholt. 4 Tage nach der letzten Immunisierung wurden die Mäuse nach Bestimmung des Antikörpertiters getötet und die Milzen für die Herstellung von Hybridomen entnommen.

D. Fusion

Nach der Vereinigung wurden die Milzzellen mit Zellen der Myelomlinie X63/Ag8.653 fusioniert. Als Fusionspromotor diente Polyethylenglykol (PEG) 1500 [50 % (Gew./Vol.) PEG 1500 in 75 mM HEPES, 5 % (Vol./Vol.) DMSO] (tropfenweise Versetzen mit PEG (1 ml) über einen Zeitraum von 1 Minute). Der Fusionsansatz wurde anschließend in serumfreiem Medium verdünnt und schonend gewaschen. Das Zellsediment wurde in HAT-Selektionsmedium resuspendiert und in einer Dichte von $2 \times 10^5$ Zellen pro ml

auf Mikrotitrationsplatten mit 96 Vertiefungen in Gegenwart von Peritonealmacrophagen ausplattiert.

Die Selektion im HAT-Medium erfolgte 7 bis 10 Tage. Unmittelbar nach Abschluß der Selektionsphase wurden die Hybridome auf HT-Medium umgestellt.

E. Selektion von Hybridomen, die M. pneumoniae-spezifische Antikörper sezernieren

Nach Ahschluß des Selektionsverfahrens in HAT-Medium wurden 100 Hybridome aus der Fusion P1 und 472 Hybridome aus der Fusion M nach dem in Beispiel 1 beschriebenen Verfahren auf die Sezernierung M. pneumoniae-spezifischer Antikörper untersucht. Insgesamt 37 Hybridome sezernierten Antikörper, die mit solubilisierten M. pneumoniae-Zellen reagierten, nicht jedoch mit den an die Festphase gebundenen Kaninchen-Serum-Antikörpern.

Die von diesen 37 Hybridomen sezernierten Antikörper wurden in ELISA-Testsystemen nach an sich bekannten Verfahren auf Reaktivität mit immobilisiertem P1-Protein, mit durch Ultraschall aufgeschlossenem Gesamtzellextrakt, mit partiell fixierten, unversehrten M. pneumoniae-Zellen und mittels Radioimmunpräzipitation auf Bindung des P1-Proteins und anderer M. pneumoniae-Oberflächenstrukturen untersucht.

Die Antikörper wurden weiterhin auf Kreuzreaktivität mit anderen Mycoplasmaarten (M. genitalium, M. fermentans, M. hominis, M. salivarium, M. orale) nach dem in den Beispielen 1 und 2 beschriebenen Verfahren untersucht. Die Kreuzreaktivität wird dabei in Prozentwerten definiert. Für die Ermittlung dieser Prozentwerte werden gleiche Mengen (100 $\mu$g/ml; 0,1 ml) der verschiedenen Mycoplasmaspezies in einer Versuchsreihe eingesetzt. Die Bindung der Antikörper an M. pneumoniae und der dadurch erhaltene Werte der Farbentwicklung werden gleich 100 % gesetzt. Die für die anderen Mycoplasmaspezies erhaltenen Werte und damit die Kreuzreaktivität werden in Relation zu diesen 100 % gesetzt.

Im Gegensatz zu den polyclonalen Kaninchen-anti-M. pneumoniae-Seren konnte für die fünf von den erfindungsgemäßen Hybridomen sezernierten monoclonalen Antikörper keine Kreuzreaktivität mit anderen Mycoplasmaspezies nachgewiesen werden.
Die Ergebnisse sind in Tabelle I zusammengefaßt.

F. Clonierung unter limitierenden Bedingungen

Die mit anderen Mycoplasmaarten nicht-kreuzreagierenden, oligoclonalen Hybridomkulturen wurden expandiert, um für die Clonierung unter limitierenden Bedingungen ausreichende Zellzahlen verfügbar zu haben.

Die Kulturen wurden dazu in der exponentiellen Wachstumsphase geerntet und die Zahl der lebenden Zellen durch Färbung mit Trypanblau, ein dem Fachmann an sich bekanntes Verfahren, bestimmt. Die Zellzahl wurde auf eine Hybridomzelle pro Vertiefung einer Mikrotitrationsplatte mit 96 Vertiefungen eingestellt und die Hybridome in Gegenwart von Peritonealmakrophagen als "Feeder-Layer" kultiviert. Nach 14 Tagen wurden die als Ergebnis der Subclonierung nunmehr monoclonalen Hybridomkulturen nach dem in den Beispielen 1 und 2 beschriebenen Verfahren auf die kontinuierliche Sezernierung von M. pneumoniae-spezifischen Antikörpern sowie deren Kreuzreaktivität mit anderen Mycoplasmaspecies untersucht.

Alle untersuchten Hybridome sezernierten Antikörper kontinuierlich über einen Zeitraum von 1 Jahr. Die Ergebnisse der Spezifitäts- bzw. Kreuzreaktivitätstests sind in Tabelle I dargestellt.

G. Die Bestimmung der Immunglobulin (Ig)-Klassen

Die Bestimmung der Ig-Klassen bzw. Ig-Subklassen erfolgte mit einem Capture-Immunoassay. Ig-Klassen-bzw. Subklassenspezifische polyclonale Antikörper wurden an Nitrocellulose-Folien gekoppelt (Fängerphase). Die aus Aszitesflüssigkeiten partiell gereinigten und Biotin-markierten monoclonalen Antikörper wurden mit der Fängerphase inkubiert. Abgefangene Antikörper wurden mit Streptavidin-Peroxidase nachgewiesen.

Die monoclonalen Antikörper P1.25 und M74 gehören der IgG1-Subklasse an, P1.27, M57 und M75 gehören der IgG3-Subklasse an; sämtliche monoclonalen Antikörper besitzen leichte Ketten des $x$-Typs.

**Beispiel 4**

**Produktion von monoclonalen Antikörpern in vivo (Aszites-Produktion)**

Das Immunsystem von weiblichen BALB/c-Mäusen im Alter zwischen 6 und 8 Wochen wurde durch intraperitoneale Injektion von 0,5 ml inkomplettem Freund'schem Adjuvans aktiviert. 24 Stunden nach dieser Injektion wurde den Tieren eine Suspension von $10^6$ Hybridomzellen eines Clons intraperitoneal appliziert. Nach der Ausbildung von Tumoren nach 2 bis 6 Wochen wurden die Tiere getötet und die Aszitesflüssigkeit durch Punktion entnommen. Der Nachweis von hohen Antikörperkonzentrationen in den Punktaten erfolgte mit dem Capture-ELISA (vgl. Beispiel 1).

**Beispiel 5**

**Spezifität der von den Hybridomen M57, M74, P1.25, P1.27 und M75 sezernierten monoclonalen Antikörper für Mycoplasma pneumonaie**

Die von den clonalen Hybridomen M25, M51, M57, M74, P1.25, P1.27 und M75 sezernierten Antikörper sowie zwei gegen M. pneumoniae gewonnene polyclonale Kaninchen-Antiseren wurden nach den in den Beispielen 1 und 2 beschriebenen Verfahren und der von dem clonalen Hybridom 11G7 sezernierten Antikörper wurde im Dot-Blot, einem an sich bekannten Verfahren, auf Spezifität für M. pneumoniae und eine mögliche Kreuzreaktivität mit anderen Mycoplasmaspezies untersucht. Das Ergebnis ist in Tabelle I dargestellt. Daraus geht hervor, daß M57, M74, P1.25, P1.27 und M75 monospezifisch für Epitope vom M. pneumoniae sind, während M25, M51, 11G7 und die beiden Kaninchen-Antiseren mit anderen "Nicht-Mycoplasma-pneumoniae-Spezies" kreuzreagieren.

**Beispiel 6**

**Avidität der von den Hybridomen M57, M74, P1.25, P1.27 und M75 sezernierten monoclonalen Antikörper für Mycoplasma pneumoniae**

Figur 1 zeigt Standardverdünnungskurven der monoclonalen Antikörper. Die Testdurchführung erfolgte wie in Beispiel 1 beschrieben, wobei die Menge der solubilisierten Zellen von Mycoplasma pneumoniae konstant 1 $\mu$g betrug und die monoclonalen Antikörper in Proben-Puffer Microbiol. wie in Figur 1 gezeigt ausverdünnt getestet wurden.

Tabelle I: Kreuzreaktivität monoklonaler Antikörper und der polyklonalen Fängerantikörper im Capture-ELISA (Antigen-ELISA)

**KREUZREAKTIVITÄT IN PROZENT**

| Erreger | monoklonale Antikörper [1] | | | | | | | | polyklonale Fängerantikörper [2] | |
|---|---|---|---|---|---|---|---|---|---|---|
| | P1.25 | M74 | P1.27 | M57 | M75 | M25 | M51 | 11G7 [3] | anti-Mpn | anti-P1 |
| M.pneumoniae | 100 | 100 | 100 | 100 | 100 | 100 | 100 | +++ | 100 | 100 |
| M.genitalium | < 1 | < 1 | < 1 | < 1 | < 1 | 18,5 | 21,2 | - | 43,7 | 0 |
| M.hominis | < 1 | < 1 | < 1 | < 1 | < 1 | 37 | 39,4 | - | 59,2 | 48 |
| M.fermentans | < 1 | < 1 | < 1 | < 1 | < 1 | 11,1 | 16,6 | - | 69 | 3,7 |
| M.salivarium | < 1 | < 1 | < 1 | < 1 | < 1 | 64,8 | 53 | | 26,3 | 55,5 |
| M.orale | < 1 | < 1 | < 1 | < 1 | < 1 | 7,4 | 10,6 | | 52,5 | 25,9 |
| H.influenzae | < 1 | < 1 | < 1 | < 1 | < 1 | < 1 | < 1 | + | < 1 | < 1 |
| B.catarrhalis | < 1 | < 1 | < 1 | < 1 | < 1 | < 1 | < 1 | +/- | < 1 | < 1 |
| N.meningitidis | < 1 | < 1 | < 1 | < 1 | < 1 | < 1 | < 1 | + | < 1 | < 1 |
| S.pneumoniae | < 1 | < 1 | < 1 | < 1 | < 1 | < 1 | < 1 | - | < 1 | < 1 |
| P.aeruginosa | < 1 | < 1 | < 1 | < 1 | < 1 | < 1 | < 1 | +/- | < 1 | < 1 |

1) im Capture-ELISA geprüft; polyklonaler Fängerantikörper: anti-M.pneumoniae-IgG
2) im homogenen polyklonalen Capture-ELISA geprüft; Fängerantikörper und Detektorantikörper (Biotin-markiert) waren identisch
3) im Dot-Blot-System geprüft; mAk 11G7 war im Capture-ELISA, unabhängig von der Spezifität des Fängerantikörpers, nicht reaktiv. Da ein Dot-Blot-Verfahren verwendet wurde, erfolgte keine Messung der optischen Dichte. Damit ist keine Berechnung der Prozentwerte möglich.

## Patentansprüche

1. Monoclonaler Antikörper und dessen Fragmente, **dadurch gekennzeichnet**, daß er (sie) spezifisch für Mycoplasma pneumoniae ist (sind) und eine Kreuzreaktivität von 1 % oder weniger mit anderen Spezies der Gattung Mycoplasma aufweist (aufweisen).

9

2. Monoclonaler Antikörper und dessen Fragmente nach Anspruch 1, **dadurch gekennzeichnet**, daß er (sie) eine Kreuzreaktivität von 1 % oder weniger mit anderen Erregerspezies der Begleitflora aufweist (aufweisen).

3. Monoclonaler Antikörper und dessen Fragmente nach Anspruch 1 oder 2, **dadurch gekennzeichnet**, daß er (sie) spezifisch für das P1-Protein von Mycoplasma pneumoniae ist (sind).

4. Monoclonaler Antikörper und dessen Fragmente nach einem der Ansprüche 1 bis 3, **dadurch gekennzeichnet**, daß die schwere Kette der $\gamma$1-Subklasse und die leichte Kette der $x$-Klasse angehört.

5. Monoclonaler Antikörper und dessen Fragmente nach einem der Ansprüche 1 bis 3, **dadurch gekennzeichnet**, daß die schwere Kette der $\gamma$3-Subklasse und die leichte Kette der $x$-Klasse angehört.

6. Hybridom, **dadurch gekennzeichnet**, daß es einen Antikörper oder dessen Fragmente nach einem der Ansprüche 1 bis 5 produziert.

7. Hybridom nach Anspruch 6, **dadurch gekennzeichnet**, daß die zur Fusion verwendete Milzzelle aus einer BALB/c-Maus stammt.

8. Hybridom nach Anspruch 6 oder 7, **dadurch gekennzeichnet**, daß die zur Fusion verwendete Myelomzelle X63/Ag8.653 ist.

9. Hybridom M57 (PHLS-Hinterlegungsnummer 91041010).

10. Hybridom M74 (PHLS-Hinterlegungsnummer 91041011).

11. Hybridom P1.25 (PHLS-Hinterlegungsnummer 91041012).

12. Hybridom P1.27 (PHLS-Hinterlegungsnummer 91041013).

13. Hybridom M75 (PHLS-Hinterlegungsnummer 91052310).

14. Kit zum Nachweis von Mycoplasma pneumoniae in einer Probe, **dadurch gekennzeichnet**, daß er mindestens einen Antikörper nach einem der Ansprüche 1 bis 5 enthält.

15. Kit nach Anspruch 14, **dadurch gekennzeichnet**, daß der Nachweis durch ein festphasenimmunometrisches System erfolgt.

16. Verfahren zur Herstellung eines Hybridoms nach einem der Ansprüche 6 bis 13, wobei man
   (a) eine Maus mit Mycoplasma pneumoniae-Zellen oder dem P1-Protein von Mycoplasma pneumoniae immunisiert;
   (b) Milzzellen aus der immunisierten Maus mit geeigneten Myelomzellen fusioniert; und
   (c) die im Schritt (b) erhaltenen Hybridome auf Sezernierung von Antikörpern, die spezifisch für M. pneumoniae sind und eine Kreuzreaktivität von 1 % oder weniger mit anderen Spezies der Gattung Mycoplasma aufweisen selektioniert.

17. Verfahren zur Herstellung eines Hybridoms nach einem der Ansprüche 6 bis 13, wobei man
   (a) eine Maus mit Mycoplasma pneumoniae-Zellen oder dem P1-Protein von Mycoplasma pneumoniae immunisiert;
   (b) Milzzellen aus der immunisierten Maus mit geeigneten Myelomzellen fusioniert;
   (c) die im Schritt (b) erhaltenen Hybridome auf Sezernierung von Antikörpern, die spezifisch für M. pneumoniae sind und eine Kreuzreaktivität von 1 % oder weniger mit anderen Spezies der Gattung Mycoplasma aufweisen selektioniert; und
   (d) die im Schritt (c) erhaltenen Hybridome auf Sezernierung von Antikörpern, die eine Kreuzreaktivität von 1 % oder weniger mit anderen Erregerspezies der Begleitflora aufweisen selektioniert.

18. Verfahren zur Herstellung eines monoclonalen Antikörpers nach einem der Ansprüche 1 bis 5, wobei man ein Hybridom nach einem der Ansprüche 6 bis 13

(a) in vitro züchtet; und

(b) den monoclonalen Antikörper aus dem Kulturmedium gewinnt.

19. Verfahren zur Herstellung eines monoclonalen Antikörpers nach einem der Ansprüche 1 bis 5, wobei man ein Hybridom nach einem der Ansprüche 6 bis 13

(a) in vivo in der Peritonealkavität einer syngenen oder congenen Maus züchtet; und

(b) den monoclonalen Antikörper aus der Ascitesflüssigkeit gewinnt.

20. Verwendung eines monoclonalen Antikörpers nach einem der Ansprüche 1 bis 5 zum Nachweis von Mycoplasma pneumoniae in einer Probe.

FIG.1